# EUROPÄISCHE PATENTSCHRIFT

(11) **EP 1 237 850 B1**
(45) Veröffentlichungstag und Bekanntmachung des Hinweises auf die Patenterteilung: **09.02.2005**
(21) Anmeldenummer: 00987274.8
(22) Anmeldetag: 20.11.2000
(51) Int. Cl.: C07C 251/48, A01N 37/50

(54) **GLYOXYLSÄUREAMIDE, VERFAHREN ZU IHRER HERSTELLUNG UND IHRE VERWENDUNG ZUR BEKÄMPFUNG VON SCHÄDLICHEN ORGANISMEN**
GLYOXYL ACID AMIDES
AMIDES D'ACIDE GLYOXYLIQUE

(30) Priorität: 02.12.1999 DE 19958165
(43) Veröffentlichungstag der Anmeldung: 11.09.2002
(73) Patentinhaber: Bayer CropScience AG, 40789 Monheim (DE)
(72) Erfinder: BOIE, Christiane, 42799 Leichlingen (DE); SEITZ, Thomas, 40764 Langenfeld (DE); HEINEMANN, Ulrich, 42799 Leichlingen (DE); FISCHER, Reiner, 40789 Monheim (DE); VAUPEL, Martin, 42799 Leichlingen (DE); WACHENDORFF-NEUMANN, Ulrike, 56566 Neuwied (DE); KUCK, Karl-Heinz, 40764 Langenfeld (DE)
(86) Internationale Anmeldenummer: PCT/EP2000/011530
(87) Internationale Veröffentlichungsnummer: WO 2001/039787

(56) Entgegenhaltungen:
- WO-A-00/41998
- WO-A-96/23763
- WO-A-98/58903
- CHEMICAL ABSTRACTS, vol. 78, no. 9, 5. März 1973 (1973-03-05) Columbus, Ohio, US; abstract no. 57925, LEMINGER, OTAKAR: "Benzene ring chemistry of alkoxylated.beta.-phenethylamines. II..beta.-Phenethylamine sulfates alkoxylated on the ring" XP002178224 & CHEM. PRUM. (1972), 22(11), 553-7 ,

## Beschreibung

Die Erfindung betrifft neue Glyoxylsäureamide, mehrere Verfahren zu ihrer Herstellung und ihre Verwendung zur Bekämpfung von schädlichen Organismen.

Es ist bereits bekannt geworden, daß bestimmte Glyoxylsäureamide die den unten beschriebenen Verbindundungen konstitutionell ähnlich sind, fungizide Eigenschaften besitzen (vergleiche z. B. WO 96/23763, WO 98/17630 oder WO 98/58903). Die fungizide Wirkung dieser Verbindungen ist jedoch in vielen Fällen unbefriedigend.

Es wurden neue Verbindungen der allgemeinen Formel (I) gefunden, 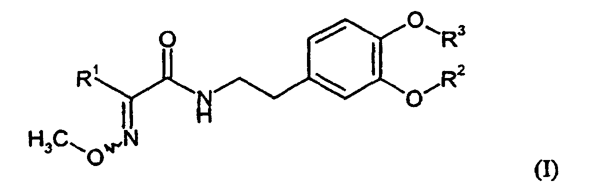 in welcher
- R¹: für Aryl steht, an das gegebenenfalls ein Cycloalkylring ankondensiert ist, wobei sowohl der Arylteil als auch der Cycloalkylteil gegebenenfalls weitere Substituenten trägt und

a)
   - R²: für Alkoxyalkyl oder gegebenenfalls durch Methyl oder Halogen substituiertes Arylalkyl steht, und
   - R³: für gegebenenfalls substituiertes Alkyl, Alkenyl oder Alkinyl steht,
   oder
b)
   - R²: für gegebenenfalls substituiertes Alkyl, Alkoxyalkyl oder Arylalkyl steht, und
   - R³: für gegebenenfalls substituiertes Alkenyl steht,
   oder
c)
   - R²: für Methyl oder Ethyl steht, und
   - R³: für gegebenenfalls substituiertes Alkyl mit mindestens 3 Kohlenstoffatomen steht,
   oder
d)
   - R²: für Alkyl mit mindestens 2 Kohlenstoffatomen steht, und
   - R³: für gegebenenfalls substituiertes Methyl oder Ethyl steht.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Aryl steht für aromatische, mono- oder polycyclische Kohlenwasserstoffringe, wie z.B. Phenyl, Naphthyl, Anthranyl, Phenanthryl, vorzugsweise Phenyl oder Naphthyl, insbesondere Phenyl.

Cycloalkyl steht für gesättigte, carbocyclische Verbindungen, die gegebenenfalls mit weiteren carbocyclischen, ankondensierten oder überbrückten Ringen ein polycyclisches Ringsystem bilden.

Cycloalkenyl steht für carbocyclische, ringförmige Verbindungen, die mindestens eine Doppelbindung enthalten und gegebenenfalls mit weiteren carbocyclischen, ankondensierten oder überbrückten Ringen ein polycyclisches Ringsystem bilden.

Weiterhin wurde gefunden, daß die neuen Glyoxylsäureamide der allgemeinen Formel (I) eine sehr gute Wirkung gegen Schadorganismen, insbesondere eine starke fungizide Wirkung zeigen.

Die erfindungsgemäßen Verbindungen liegen gegebenenfalls als Mischungen verschiedener möglicher isomerer Formen, insbesondere von Stereoisomeren, wie z.B. E- und Z-, cis- oder trans-, threo- und erythro-, sowie optischen Isomeren vor. Es werden sowohl die E- als auch die Z-Isomeren, wie auch die threo- und erythro-, sowie die optischen Isomeren sowie beliebige Mischungen dieser Isomeren beschrieben und beansprucht.

Gegenstand der Erfindung sind vorzugsweise Verbindungen der Formel (I), in welcher
- R¹: für Aryl steht, an das gegebenenfalls ein Cycloalkylring mit 3 bis 10 Ringgliedern ankondensiert ist, wobei der Cycloalkylteil gegebenenfalls durch 1 bis 4 Alkylketten mit jeweils 1 - 4 Kohlenstoffatomen substituiert ist und der Arylteil gegebenenfalls zusätzlich durch die nachstehend aufgezählten Substituenten substituiert ist:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halo-genalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen, und

a)
   - R²: für Alkoxyalkyl mit insgesamt 2 bis 10 Kohlenstoffatomen oder gegebenenfalls durch Methyl oder Halogen substituiertes Arylalkyl mit 1 bis 4 Kohlenstoffatomen im Alkylteil steht und
   - R³: für gegebenenfalls durch Cyano, Alkoxy oder Alkoxycarbonyl, substituiertes Alkyl mit 1 bis 8 Kohlenstoffatomen, Alkenyl oder Alkinyl mit 2 bis 8 Kohlenstoffatomen steht,
   oder
b)
   - R²: für Alkyl mit 1 bis 8 Kohlenstoffatomen oder gegebenenfalls durch Methyl oder Halogen substituiertes Arylalkyl mit 1 bis 4 Kohlenstoffatomen im Alkylteil steht und
   - R³: für gegebenenfalls durch Cyano, Alkoxy oder Alkoxycarbonyl substituiertes Alkenyl mit 2 bis 8 Kohlenstoffatomen steht,
   oder
c)
   - R²: für Methyl oder Ethyl steht, und
   - R³: für gegebenenfalls durch Cyano, Alkoxy oder Alkoxycarbonyl substituiertes Alkyl mit mindestens 3 Kohlenstoffatomen steht,
   oder
d)
   - R²: für Alkyl mit mindestens 2 Kohlenstoffatomen steht, und
   - R³: für gegebenenfalls durch Cyano, Alkoxy oder Alkoxycarbonyl substituiertes Methyl oder Ethyl steht.

Die Erfindung betrifft insbesondere Verbindungen der Formel (I), in welcher
- R¹: für jeweils gegebenenfalls einfach bis dreifach substituiertes Phenyl steht, an das gegebenenfalls ein Cyclopropyl-, Cyclobutyl-, Cyclopentyl-, Cyclohexyl-, Cycloheptyl- Cyclooctyl- oder Cyclononylring ankondensiert ist, wobei der Cycloalkylteil gegebenenfalls durch einfach bis vierfach durch Methyl, Ethyl, n- oder i-Propyl, n-, i-, s- oder t-Butyl substituiert ist und der Phenylteil gegebenenfalls zusätzlich durch die nachstehend aufgezählten Substituenten substituiert ist:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl, Cyclopropyl, Cyclobutyl, Cyclopentyl oder Cyclohexyl, und

a)
   - R²: für Methoxyethyl, Ethoxyethyl oder gegebenenfalls durch Methyl, Fluor oder Chlor substituiertes Benzyl oder Phenethyl steht und
   - R³: für gegebenenfalls durch Cyano, Methoxy, Ethoxy, Methoxycarbonyl oder Ethoxycarbonyl substituiertes Methyl, Ethyl, n- oder i-Propyl, n-, i-, s- oder t-Butyl, Pentyl, Hexyl, Heptyl, Octyl, Allyl, Methylallyl, Crotonyl, Propinyl, Butinyl oder Benzyl steht,
   oder
b)
   - R²: für Methyl, Ethyl, n- oder i-Propyl, n-, i-, s- oder t-Butyl, Pentyl, Hexyl, Heptyl oder Octyl oder gegebenenfalls durch Methyl, Fluor oder Chlor substituiertes Benzyl oder Phenethyl steht, und
   - R³: für gegebenenfalls durch Cyano, Methoxy, Ethoxy, Methoxycarbonyl oder Ethoxycarbonyl substituiertes Allyl, Methylallyl, Crotonyl, steht,
   oder
c)
   - R²: für Methyl oder Ethyl steht, und
   - R³: für gegebenenfalls durch Cyano, Methoxy, Ethoxy, Methoxycarbonyl oder Ethoxycarbonyl substituiertes n- oder i-Propyl, n-, i-, s- oder t-Butyl, Pentyl, Hexyl, Heptyl oder Octyl steht,
   oder
d)
   - R²: für n- oder i-Propyl, n-, i-, s- oder t-Butyl, Pentyl, Hexyl, Heptyl oder Octyl steht, und
   - R³: für gegebenenfalls durch Cyano, Methoxy, Ethoxy, Methoxycarbonyl oder Ethoxycarbonyl substituiertes Methyl oder Ethyl steht.

R¹ steht besonders bevorzugt für para-Chlorphenyl, para-Bromphenyl, 1,2,3,4-Tetrahydronaphthalin oder für Indan.

R² steht besonders bevorzugt für Ethyl, insbesondere Methyl.

R³ steht besonders bevorzugt für Cyanomethyl, Allyl oder Propargyl.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen für diese Reste angegebenen Restedefinitionen werden unabhängig von der jeweilig angegebenen Kombination der Reste, beliebig auch durch Restedefinitionen anderer Vorzugsbereiche ersetzt.

Schließlich wurde gefunden, daß man die neuen Glyoxylsäureamide der allgemeinen Formel (I) erhält, wenn man
a) Carbonsäurederivate der allgemeinen Formel (II), 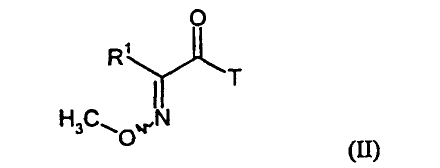
in welcher
- R¹: die oben angegebene Bedeutung hat und
- T: für Hydroxy, Halogen oder Alkoxy steht,
mit einem Amin der allgemeinen Formel (III) 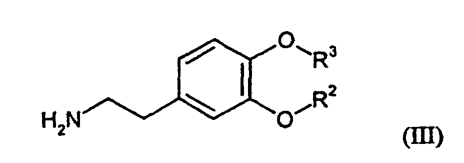 in welcher
R² und R³ die oben angegebenen Bedeutungen haben,
- oder mit einem Säureadditionskomplex hiervon -
gegebenenfalls in Gegenwart eines Säureakzeptors, gegebenenfalls in Gegenwart eines Kondensationsmittels und gegebenenfalls in Gegenwart eines Verdünnungsmittels umsetzt.

Schließlich wurde gefunden, daß die neuen Verbindungen der Formel (I) eine sehr starke Wirkung gegen schädliche Organismen.

Die zur Durchführung des erfindungsgemäßen Verfahrens a) als Ausgangsstoffe benötigten Carbonsäurederivate sind durch die Formel (II) allgemein definiert. In dieser Formel (II) hat R¹ vorzugsweise, bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für R¹ angegeben wurde; T steht vorzugsweise für Alkoxy mit 1 bis 4 Kohlenstoffatomen, insbesondere für Methoxy oder Ethoxy, für Hydroxy oder Chlor.

Die Ausgangsstoffe der Formel (II) sind bekannt und/oder können nach an sich bekannten Verfahren hergestellt werden (vgl. EP-A 493 711, EP-A 432 503, DE-A 3 938 054, J. Heterocycl. Chem. (1990), 27(3), 487-95, Farmaco, Ed. Sci. (1980), 35(5), 394-404, Justus Liebigs Ann. Chem. (1969), 722, 38-44, Justus Liebigs Ann. Chem. (1969), 722, 29-37, Tetrahedron 1971, 3431-6, DE 222 3375).

Die weiterhin zur Durchführung des erfindungsgemäßen Verfahrens a) als Ausgangsstoffe benötigten Amine sind durch die Formel (III) allgemein definiert. In dieser Formel (III) haben R² und R³ vorzugsweise, bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für R² und R³ angegeben wurden.

Die Amine der Formel (III) sind teilweise bekannte organische Synthesechemikalien und/oder können nach an sich bekannten Verfahren hergestellt werden.

Neu und ebenfalls Gegenstand der vorliegenden Anmeldung sind Amine der Formel (III-a), 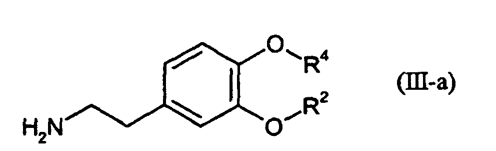 in welcher
- R²: die oben angegebene Bedeutung gemaß Fall a) hat und
- R⁴: für Propargyl oder Cyanomethyl steht.

Die Amine der Formel (III-a) werden erhalten (Verfahren b), wenn man Hydroxyverbindungen der allgemeinen Formel (IV), 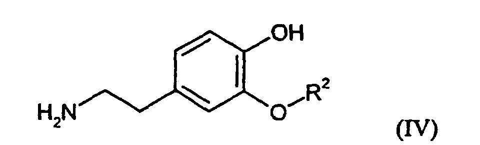 in welcher
- R²: die oben angegebene Bedeutung hat,
mit Allyl-, Propargyl-chlorid, -bromid oder -iodid oder Chlor- Brom- oder Iodacetonitril, gegebenenfalls in Gegenwart eines Verdünnungsmittels, wie beispielsweise Acetonitril, und gegebenenfalls in Gegenwart eines Säureakzeptors, wie beispielsweise Kaliumcarbonat, umsetzt.

Die Aminogruppe der Verbindungen der Formel (TV) wird vor der Umsetzung gegebenenfalls mit einer für Amine üblichen Schutzgruppe, wie beispielsweise t-Butoxycarbonyl, nach üblichen Methoden versehen. Hierbei entstehen Verbindungen der Formel (IV*), 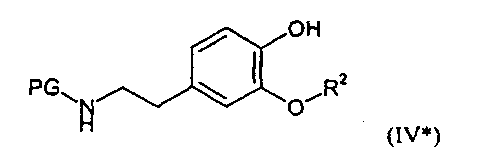 in welcher
- R²: die oben angegebene Bedeutung hat und
- PG: für die Schutzgrupe steht,
die nach der Umsetzung, die zunächst zu Verbindungen der Formel (II-a*), 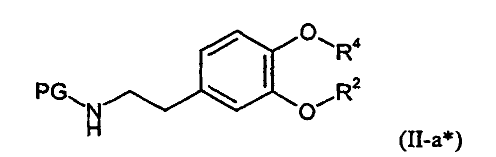 in welcher
- R², R⁴ und PG: die oben angegebene Bedeutung haben,
führt, wieder nach literaturbekannten Methoden abgespalten wird (siehe auch die Herstellungsbeispiele). Abhängig von der Aufarbeitung entstehen die freien Amine oder deren Salze, beispielsweise Hydrochloride.

Die zur Durchführung des erfindungsgemäßen Verfahrens b) als Ausgangsstoffe benötigten Hydroxyverbindungen sind durch die Formel (IV) allgemein definiert. In dieser Formel (IV) hat R² vorzugsweise, bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für R² angegeben wurde.

Die Hydroxyverbindungen der Formel (IV) sind handelsübliche Synthesechemikalien oder können nach bekannten Methoden erhalten werden (vergleiche z. B. J. Chem. Soc. 127 (1925), 560 und J. Amer. Chem. Soc. 72 (1950), 2781).

Die zur Durchführung des erfindungsgemäßen Verfahrens b) weiterhin als Ausgangsstoffe benötigten Verbindungen Allyl-, Propargyl-chlorid, -bromid oder -iodid oder Chlor- Brom- oder Iodacetonitril sind allgemein übliche Synthesechemikalien. Das erfindungsgemäße Verfahren a) wird gegebenenfalls in Gegenwart eines Verdünnungsmittels durchgeführt. Als solche kommen Wasser und organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykol-dimethyl- oder -diethylether; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester.

Das erfindungsgemäße Verfahren a) wird gegebenenfalls in Gegenwart eines geeigneten Säureakzeptors durchgeführt. Als solche kommen alle üblichen anorganischen oder organischen Basen infrage. Hierzu gehören beispielsweise Erdalkalimetall- oder Alkalimetallhydroxide, -alkoholate, -acetate, -carbonate oder -hydrogencarbonate, wie beispielsweise Natriummethylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat, oder Natriumhydrogencarbonat, sowie tertiäre Amine, wie Trimethylamin, Triethylamin, Tributylamin, N,N-Dimethylanilin, N,N-Dimethyl-benzylamin, Pyridin, N-Methylpiperidin, N,N-Dimethylaminopyridin, Diazabicyclooctan (DABCO), Diazabicyclononen (DBN) oder Diazabicycloundecen (DBU).

Das erfindungsgemäße Verfahren a) wird gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ) oder Triphenylphosphin/Tetrachlorkohlenstoff.

Das erfindungsgemäße Verfahren a) wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung des erfindungsgemäßen Verfahrens a) in einem größeren Bereich variiert werden. Im allgemeinen arbeitet man bei Temperaturen zwischen -50°C und +150°C, vorzugsweise bei Temperaturen zwischen -20°C und 150°C.

Zur Durchführung des erfindungsgemäßen Verfahrens a) setzt man je Mol an Carbonsäurederivat der Formel (II) im allgemeinen 1 bis 5 Mol, vorzugsweise 1,0 bis 2,5 Mol an Amin ein.

Die Reaktionsdurchführung, Aufarbeitung und Isolierung der Reaktionsprodukte erfolgt nach bekannten Verfahren (vgl. die Herstellungsbeispiele).

Das erfindungsgemäße Verfahren a) kann auch als zweistufiger Prozess durchgeführt werden. Dabei werden die Carbonsäurederivate der allgemeinen Formel (II) zunächst in eine aktivierte Form überführt und in einem anschließenden Schritt mit den Aminen der allgemeinen Formel (III) zu den erfindungsgemäßen Glyoxylsäureamiden der allgemeinen Formel (I) umgesetzt.

Als aktivierte Form der Carbonsäurederivate der Formel (II) kommen alle Carboxyaktivierten Derivate infrage, wie z.B. Säurehalogenide, bevorzugt Säurechloride, Säureazide, ferner symmetrische und gemischte Anhydride, wie beispielsweise die gemischten o-Alkylkohlensäureanhydride, weiterhin aktivierte Ester, wie z.B. p-Nitrophenylester oder N-Hydroxisuccinimidester sowie Addukte mit Kondensationsmitteln, wie z.B. Dicyclohexylcarbodiimid oder in situ erzeugte aktivierte Formen der Carbonsäuren.

Die erfindungsgemäßen Verfahren werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die Reaktionsdurchführung, Aufarbeitung und Isolierung der Reaktionsprodukte erfolgt nach bekannten Verfahren.

Die erfindungsgemäßen Stoffe weisen eine starke mikrobizide Wirkung auf und können zur Bekämpfung von unerwünschten schädlichen Organismen, insbesondere Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

In bestimmten Konzentrationen zeigen die erfindungsgemäßen Verbindungen auch eine herbizide oder insektizide Wirkung.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae; Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicuiaria-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Altemaria-Arten, wie beispielsweise Altemaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Methoden oder Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Dabei lassen sich die erfindungsgemäßen Wirkstoffe mit besonders gutem Erfolg zur Bekämpfung von Krankheiten im Wein-, Obst- und Gemüseanbau, wie beispielsweise gegen Alternaria-, Phytophtora- und Plasmopara-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Verbindungen können bei allen Pflanzen und Pflanzensorten, einschließlich transgenen Pflanzen und Pflanzensorten verwendet werden, wobei bei transgenen Pflanzen und Pflanzensorten auch synergistische Effekte auftreten können.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampmpylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpictonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB), Quimomethionate
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthatenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl)-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O)-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl)-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazalylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin-Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Dicofol, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Isazofos, Isofenphos, Isoxathion, Ivermectin,
Kernpolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazo]
2-(Acetlyoxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid
3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon
Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-ylester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat
N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl)-N'-methyl-N"-nitro-guanidin
N-Methyl-N'-(1-methyl-2-propenyl)-1,2-hydrazindicarbothtoamid
N-Methyl-N'-2-propenyl-1,2-hydrazindicarbothioamid
O,O-Diethyl-[2-(dipropylamino)-2-oxoethyl]-ethylphosphoramidothioat

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sproßpilze, Schimmel und diphasische Pilze (z.B. gegen Candida-Spezies wie Candida albicans, Candida glabrata) wie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfaßbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die nachfolgenden Beispiele dienen der Erläuterung der Erfindung. Die Erfindung ist jedoch nicht auf die Beispiele limitiert.

### Herstellungsbeispiele

### Beispiel 1

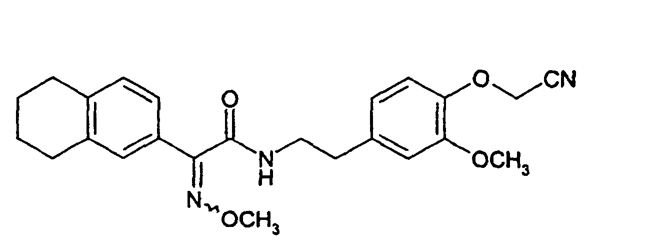

### Verfahren a)

Zu einer Lösung von 0,94 g (4,0 mMol) 2-(Methoxyimino)-2-(5,6,7,8-tetrahydro-2-naphthyl)essigsäure in 15 ml Dichlormethan und 0,5 ml Dimethylformamid gibt man 2,2 ml (4,4 mMol) einer 2-molaren Lösung von Oxalylchlorid in Dichlormethan und rührt 2 Stunden bei Raumtemperatur. Die Mischung wird bei vermindertem Druck eingeengt und in 15 ml Tetrahydrofuran gelöst. Diese Lösung gibt man in eine auf 0°C gekühlte Lösung von 1,37 g (4,0 mMol) 2-[4-(Cyanomethoxy)-3-methoxyphenyl]-1-ethanaminiumchlorid und 1,2 ml (8,6 mMol) Triethylamin in 30 ml Tetrahydrofuran. Man rührt 18 Stunden ohne weitere Kühlung und filtriert den Niederschlag ab. Das Filtrat wird eingeengt, in 200 ml Dichlormethan aufgenommen, mit Wasser gewaschen, über Natriumsulfat getrocknet und wiederum eingeengt. Der Rückstand wird mit Petrolether/Essigester (10:1) an Kieselgel chromatografiert. Man erhält 1,3 g (77 % der Theorie) N-[4-(Cyanomethoxy)-3-methoxyphenethyl]-2-(methoxyimino)-2-(5,6,7,8-tetrahydro-2-naphthyl)acetamid als Isomerengemisch.

### HPLC:

Isomer A: logP = 3.16
Isomer B: logP = 3.41

Die Bestimmung der logP-Werte erfolgte gemäß EEC-Directive 79/831 Annex V. A8 durch HPLC (Gradientenmethode, Acetonitril/0,1 % wäßrige Phosphorsäure)

Analog Beispiel 1, sowie entsprechend der allgemeinen Beschreibung der erfindungsgemäßen Herstellungsverfahren, können auch die in der nachstehenden Tabelle 1 aufgeführten Verbindungen der Formel (I) hergestellt werden:

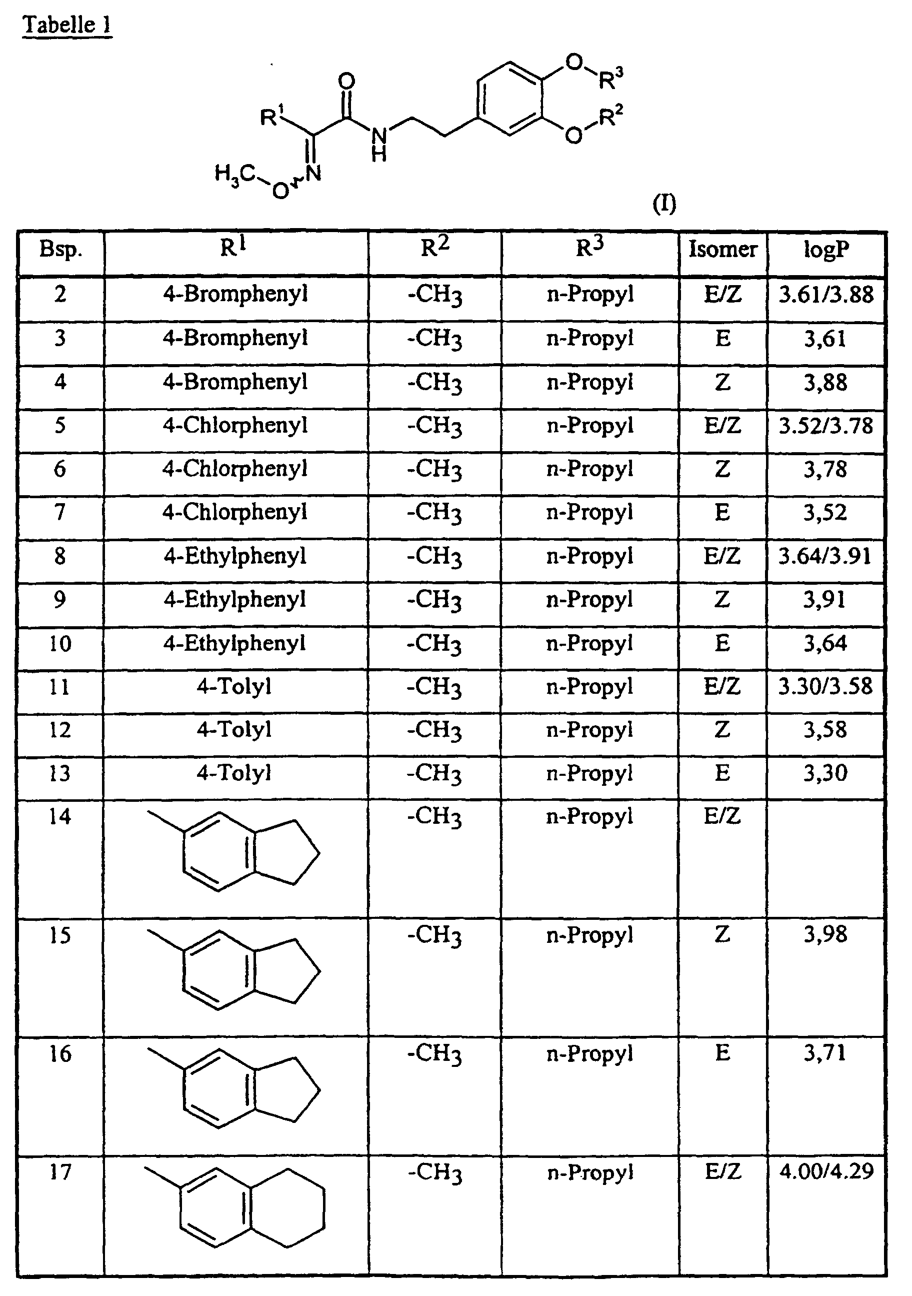

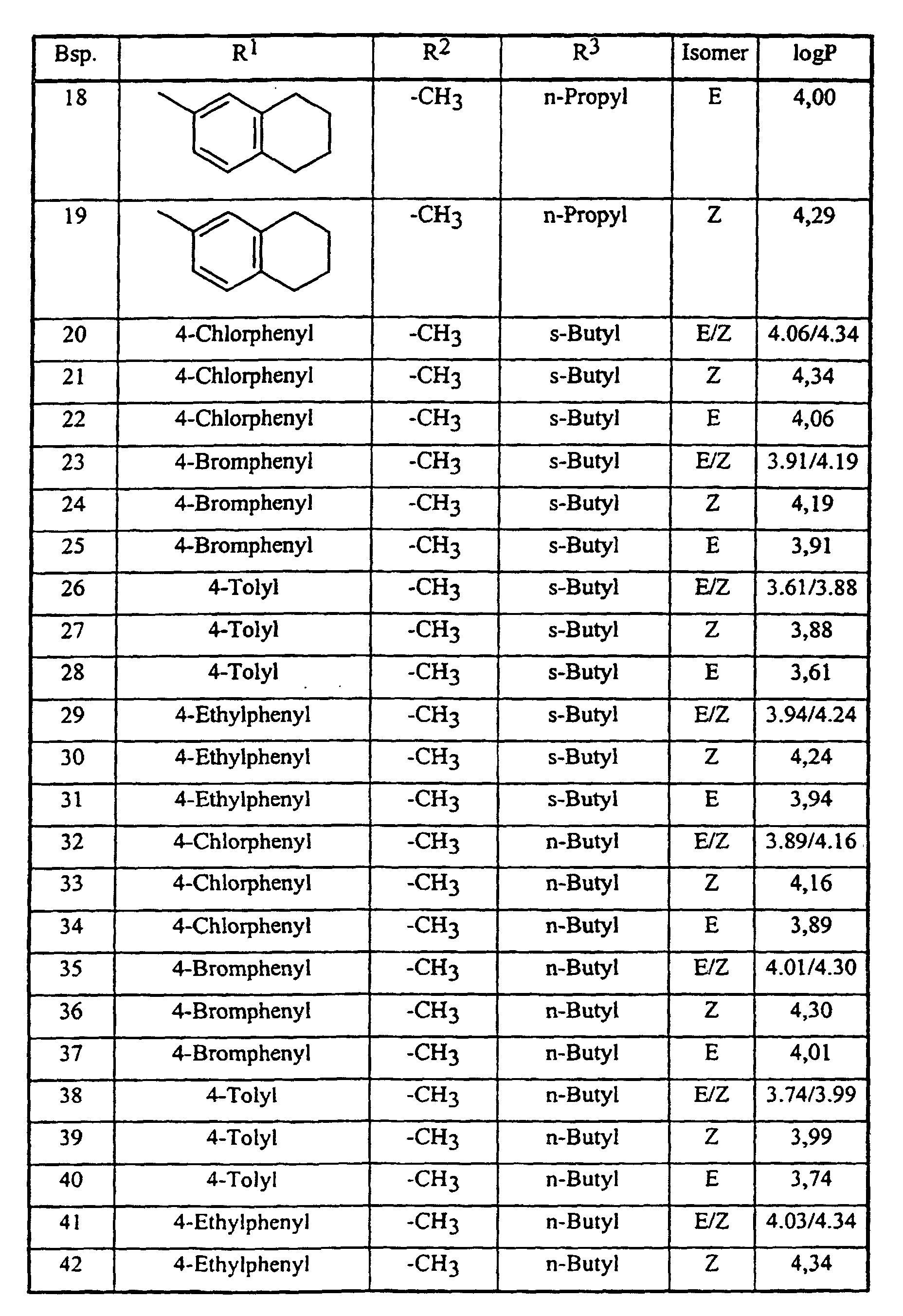

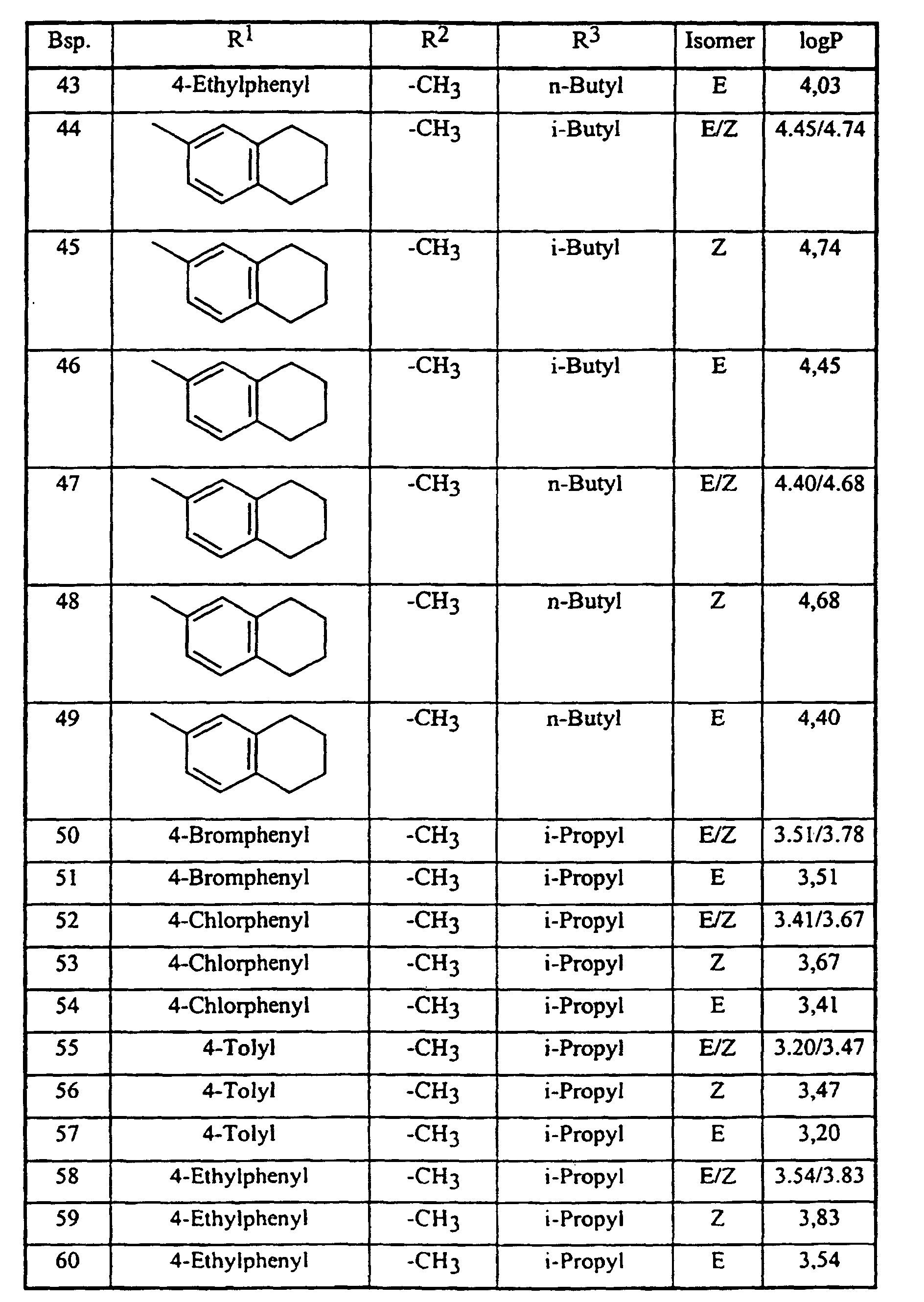

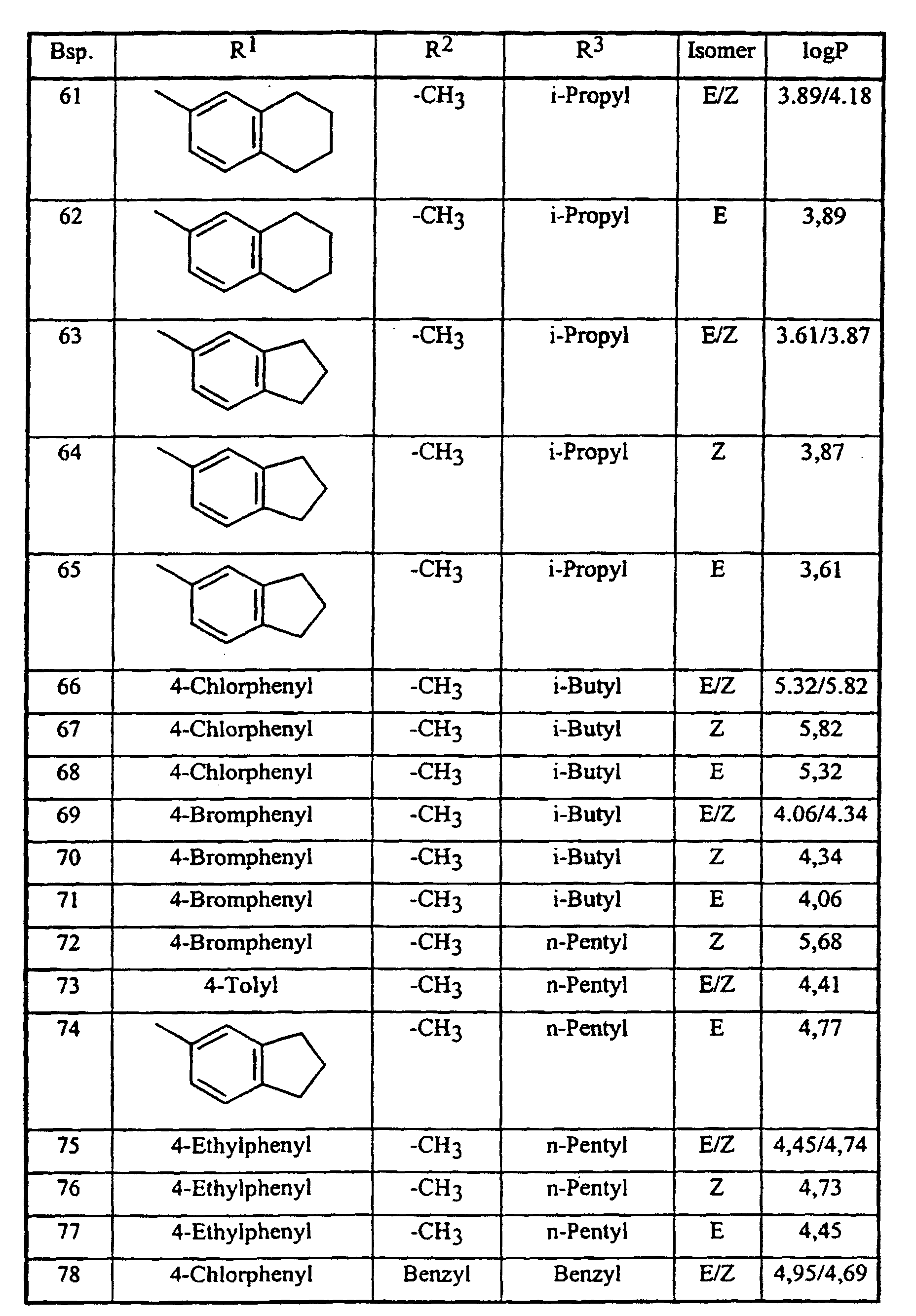

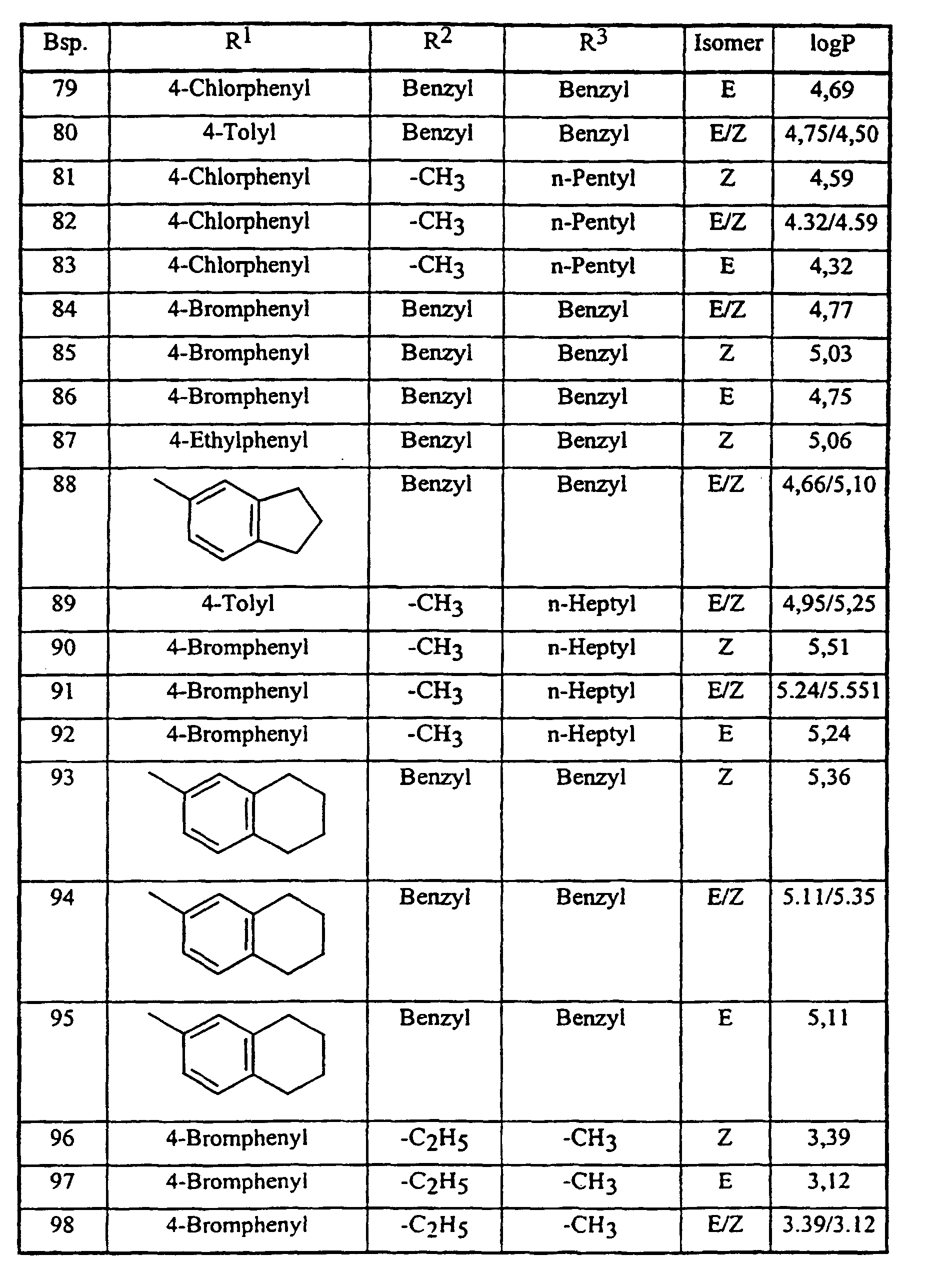

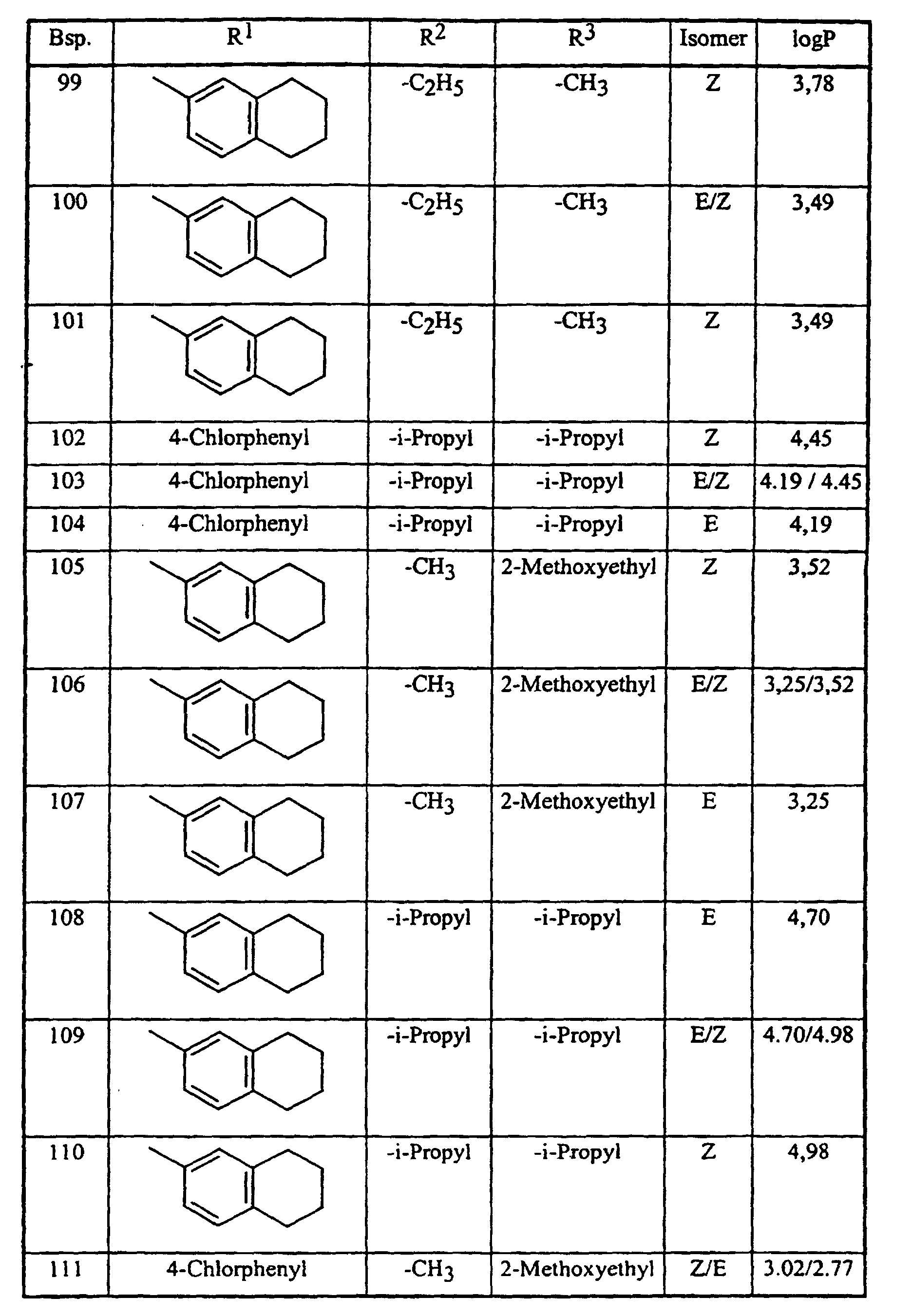

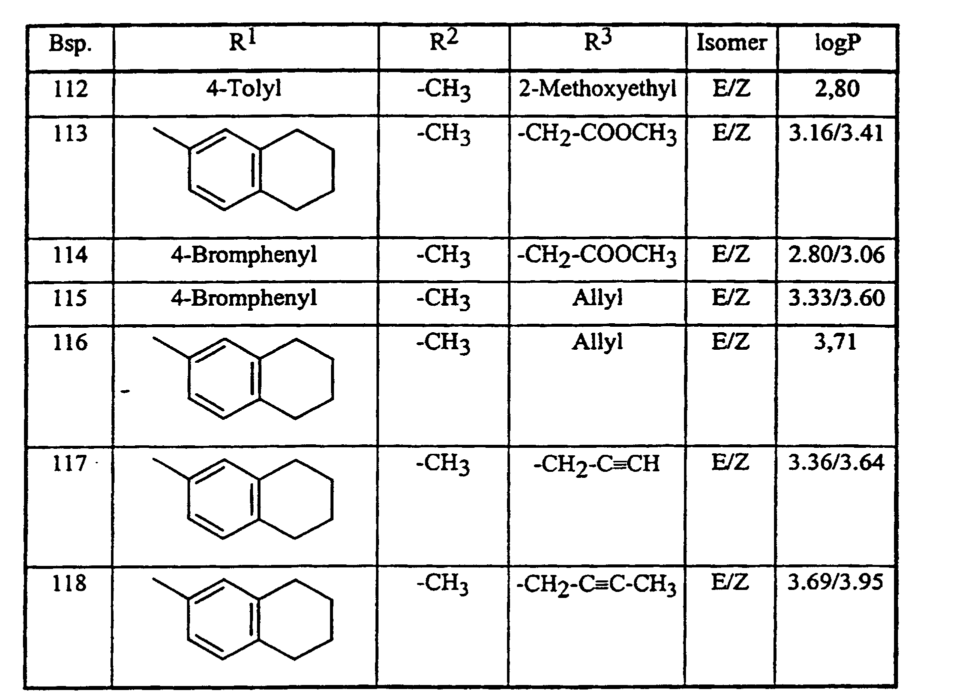

Die Bestimmung der logP-Werte erfolgte gemäß EEC-Directive 79/831 Annex V. A8 durch HPLC (Gradientenmethode, Acetonitril/0,1 % wäßrige Phosphorsäure)

### Herstellung der Amine der Formel (II-a):

### Beispiel (II-a-1)

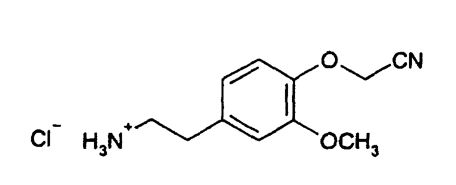

### Verfahren b (unter Verwendung einer Schutzgruppe)

Zu einer Lösung von 6,2 g (30,4 mMol) 4-Hydroxy-3-methoxyphenethylamin Hydrochlorid in 50 ml Essigsäureethylester und 5 ml Triethylamin gibt man 7,0 g (32 mMol) Pyrokohlensäure-di-tert.-butylester und rührt 18 Stunden bei Raumtemperatur. Anschließend gibt man 100 ml Essigsäureethylester zu, wäscht mit 50 ml Wasser dann mit 50 ml verdünnter Zitronensäure und 50 ml Natriumhydrogencarbonatlösung und schließlich mit 50 ml gesättigter Natriumchloridlösung. Die organische Phase wird über Natriumsulfat getrocknet und bei vermindertem Druck eingeengt.

Man erhält 7,8 g (96 % der Theorie) 4-Hydroxy-3-methoxyphenethylcarbaminsäuretert.-butylester (Verbindung IV*-1)
HPLC: logP = 2,09

Zu einer Lösung von 2,4 g (9,0 mMol) 4-Hydroxy-3-methoxyphenethylcarbaminsäure-tert.-butylester (Verbindung IV*-1) in 20 ml Aceton werden 1,7 g (22,5 mMol) Chloracetonitril, 3 g wasserfreies Kaliumcarbonat und 0,1 g Kaliumiodid gegeben und 18 Stunden unter Rückfluß erhitzt. Das Reaktionsgemisch wird auf 100 ml Wasser gegeben und mit 200 ml Ether extrahiert. Die organische Phase wird zweimal mit 10%iger Natronlauge gewaschen, über Natriumsulfat getrocknet und eingeengt. Man erhält 2,0 g (73 % der Theorie) 4-(Cyanomethoxy)-3-methoxyphenethylcarbaminsäure-tert.-butylester (Verbindung II-a*-1).
(HPLC: logP = 2,51).

Zu einer Lösung von 2,0 g (6,5 mMol) 4-(Cyanomethoxy)-3-methoxyphenethylcarbaminsäure-tert.-butylester (Verbindung II-a*-I) in 10 ml Essigsäureethylester gibt man 3 ml einer gesättigten etherischen Chlorwasserstofflösung rührt 18 Stunden und filtriert den entstandenen Niederschlag ab. Dieser wird zweimal mit Essigsäureethylester gewaschen und im Vakuum getrocknet. Man erhält 1,6 g (73 % der Theorie) 2-[4-(Cyanomethoxy)-3-methoxyphenyl]-1-ethanaminium chlorid.
(HPLC: logP = 0,10).

Analog Beispiel (II-a-1), sowie entsprechend der allgemeinen Beschreibung der erfindungsgemäßen Herstellungsverfahren, können auch die in der nachstehenden Tabelle 2 aufgeführten Verbindungen der Formel (I) hergestellt werden:

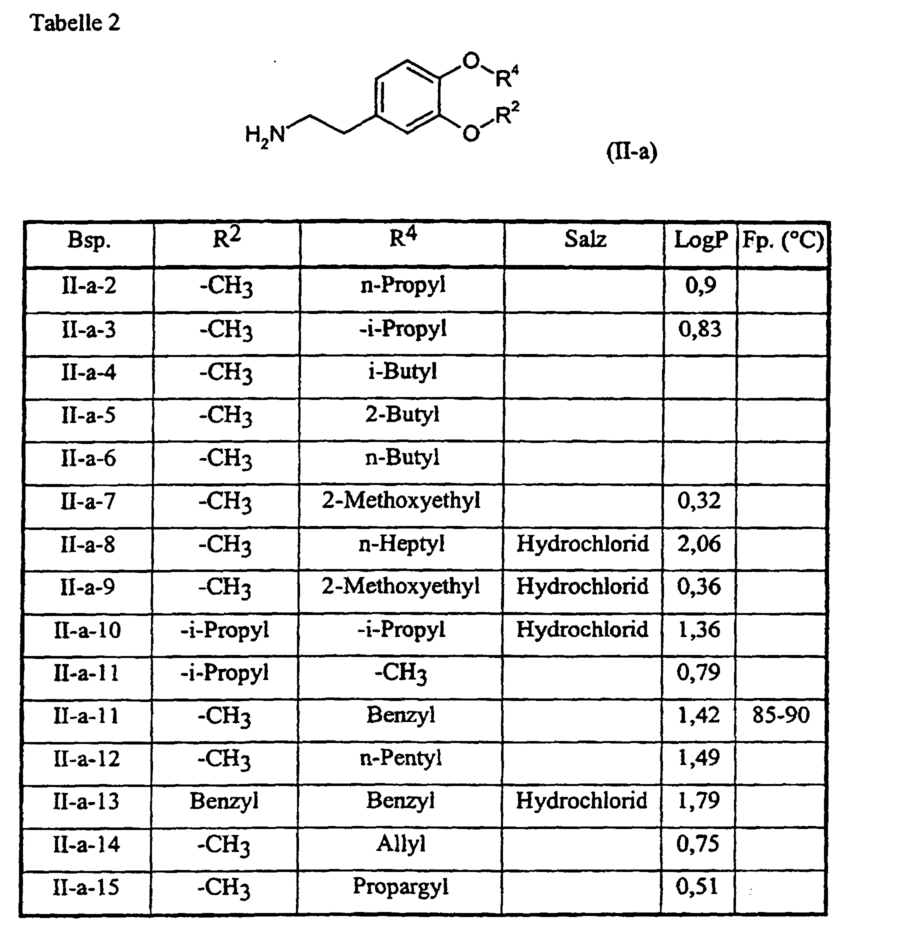

Die Bestimmung der logP-Werte erfolgte gemäß EEC-Directive 79/831 Annex V. A8 durch HPLC (Gradientenmethode, Acetonitril/0,1 % wäßrige Phosphorsäure).

### Anwendungsbeispiele:

### Beispiel A

Phytophthora-Test (Tomate) / protektiv
- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Phytophthora infestans inokuliert. Die Pflanzen werden dann in einer Inkubationskabine bei ca. 20°C und 100 % relativer Luftfeuchtigkeit aufgestellt.

3 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen ( 3, 52, 57, 60, 61, 62, 63, 65, 107, 113, 115, 116) aufgeführten erfindungsgemäßen Stoffe bei einer Aufwandmenge von 50 g/ha einen Wirkungsgrad von 89 % oder mehr.

### Beispiel B

Plasmopara-Test (Rebe) / protektiv
- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Plasmopara viticola inokuliert und verbleiben dann 1 Tag in einer Inkubationskabine bei ca. 20°C und 100 % relativer Luftfeuchtigkeit. Anschließend werden die Pflanzen 5 Tage im Gewächshaus bei ca. 21°C und ca. 90 % relativer Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen ( 52, 60, 63, 65, 100, 111, 113, 115, 116) aufgeführten erfindungsgemäßen Stoffe bei einer Aufwandmenge von 50 g/ha einen Wirkungsgrad von 83 % oder mehr.

### Beispiel C

Altemaria-Test (Tomate) / protektiv
- Lösungsmittel:: 50 Gewichtsteile N,N-Dimethylformamid
- Emulgator:: 1,2 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit werden junge Tomatenpflanzen mit der Wirkstoffzubereitung in der angegebenen Aufwandmenge besprüht. Ein Tag anch der Behandlung werden die Pflanzen mit einer Sporensuspension von Altemaria solani inokuliert. Anschließend stehen die Pflanzen bei 100% rel. Luftfeuchtigkeit und einer Temperatur von 20°C.

3 Tage nach der Inokulation erfolgt die Auswertung. Dabei bedeutet 0% ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (45, 46, 48, 49) aufgeführten erfindungsgemäßen Stoffe bei einer Aufwandmenge von 750 g/ha einen Wirkungsgrad von 90 % oder mehr.

## Patentansprüche

1. Verbindungen der allgemeinen Formel (I), 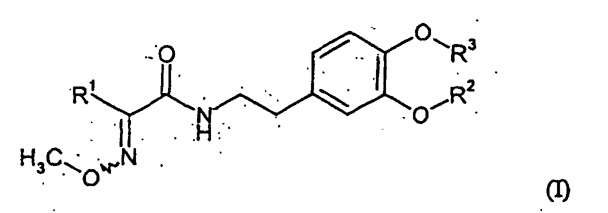 in welcher
R¹ für Aryl steht, an das gegebenenfalls ein Cycloalkylring ankondensiert ist, wobei sowohl der Arylteil als auch der Cycloalkylteil gegebenenfalls weitere Substituenten trägt und
a)
R² für Alkoxyalkyl oder gegebenenfalls durch Methyl oder Halogen substituiertes Arylalkyl steht, und
R³ für gegebenenfalls substituiertes Alkyl, Alkenyl oder Alkinyl steht,
oder
b)
R² für gegebenenfalls substituiertes Alkyl, Alkoxyalkyl oder Arylalkyl steht, und
R³ für gegebenenfalls substituiertes Alkenyl steht,
oder
c)
R² für Methyl oder Ethyl steht, und
R³ für gegebenenfalls substituiertes Alkyl mit mindestens 3 Kohlenstoffatomen steht,
oder
d)
R² für Alkyl mit mindestens 2 Kohlenstoffatomen steht, und
R³ für gegebenenfalls substituiertes Methyl oder Ethyl steht.

2. Verbindungen der Formel (I), gemäß Anspruch 1, **dadurch gekennzeichnet, dass**
R¹ für Aryl steht, an das gegebenenfalls ein Cycloalkylring mit 3 bis 10 Ringgliedern ankondensiert ist, wobei der Cycloalkylteil gegebenenfalls durch 1 bis 4 Alkylketten mit jeweils 1 - 4 Kohlenstoffatomen substituiert ist und der Arylteil gegebenenfalls zusätzlich durch die nachstehend aufgezählten Substituenten substituiert ist: Halogen, Cyano, Nitro, Amino, Hydroxy, Formyl, Carboxy, Carbamoyl, Thiocarbamoyl;
jeweils geradkettiges oder verzweigtes Alkyl, Alkoxy; Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen, und
a)
R² für Alkoxyalkyl mit insgesamt 2 bis 10 Kohlenstoffatomen oder gegebenenfalls durch Methyl oder Halogen substituiertes Arylalkyl mit 1 bis 4 Kohlenstoffatomen im Alkylteil steht und
R³ für gegebenenfalls durch Cyano, Alkoxy oder Alkoxycarbonyl, substituiertes Alkyl mit 1 bis 8 Kohlenstoffatomen, Alkenyl oder Alkinyl steht,
oder
b)
R² für Alkyl mit 1 bis 8 Kohlenstoffatomen oder gegebenenfalls durch Methyl oder Halogen substituiertes Arylalkyl mit 1 bis 4 Kohlenstoffatomen im Alkylteil steht und
R³ für gegebenenfalls durch Cyano, Alkoxy oder Alkoxycarbonyl substituiertes Alkenyl mit 2 bis 8 Kohlenstoffatomen steht,
oder
c)
R² für Methyl oder Ethyl steht, und
R³ für gegebenenfalls durch Cyano, Alkoxy oder Alkoxycarbonyl substituiertes Alkyl mit mindestens 3 Kohlenstoffatomen steht,
oder
'd)
R² für Alkyl mit mindestens 2 Kohlenstoffatomen steht, und
R³ für gegebenenfalls durch Cyano, Alkoxy oder Alkoxycarbonyl substituiertes Methyl oder Ethyl steht.

3. Verbindungen der Formel (I), gemäß Anspruch, 1, **dadurch gekennzeichnet, dass**
R¹ für jeweils gegebenenfalls einfach bis dreifach substituiertes Phenyl steht, an das gegebenenfalls ein Cyclopropyl-, Cyclobutyl-, Cyclopentyl-, Cyclohexyl-, Cycloheptyl- Cyclooctyl- oder Cyclononylring ankondensiert ist, wobei der Cycloalkylteil gegebenenfalls durch einfach bis vierfach durch Methyl, Ethyl, n- oder i-Propyl, n-, i-, s- oder t-Butyl substituiert ist und der Phenylteil gegebenenfalls zusätzlich durch die nachstehend aufgezählten Substituenten substituiert ist:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl, Cyclopropyl, Cyclobutyl, Cyclopentyl oder Cyclohexyl, und
a)
R² für Methoxyethyl, Ethoxyethyl oder gegebenenfalls durch Methyl, Fluor oder Chlor substituiertes Benzyl oder Phenethyl steht und
R³ für gegebenenfalls durch Cyano, Methoxy, Ethoxy, Methoxycarbonyl oder Ethoxycarbonyl substituiertes Methyl, Ethyl, n- oder i-Propyl, n-, i-, s- oder t-Butyl, Pentyl, Hexyl, Heptyl, Octyl, Allyl, Methylallyl, Crotonyl, Propinyl, Butinyl oder Benzyl steht,
oder
b)
R² für Methyl, Ethyl, n- oder i-Propyl, n-, i-, s- oder t-Butyl, Pentyl, Hexyl, Heptyl oder Octyl oder gegebenenfalls durch Methyl, Fluor oder Chlor substituiertes Benzyl oder Phenethyl steht, und
R³ für gegebenenfalls durch Cyano, Methoxy, Ethoxy, Methoxycarbonyl oder Ethoxycarbonyl substituiertes Allyl, Methylallyl, Crotonyl, steht,
oder
c)
R² für Methyl oder Ethyl steht, und
R³ für gegebenenfalls durch Cyano, Methoxy, Ethoxy, Methoxycarbonyl oder Ethoxycarbonyl substituiertes n- oder i-Propyl, n-, i-, s- oder t-Butyl, Pentyl, Hexyl, Heptyl oder Octyl steht,
oder
d)
R² für n- oder i-Propyl, n-, i-, s- oder t-Butyl, Pentyl, Hexyl, Heptyl oder Octyl steht, und
R³ für gegebenenfalls durch Cyano, Methoxy, Ethoxy, Methoxycarbonyl öder Ethoxycarbonyl substituiertes Methyl oder Ethyl steht.

4. Verbindungen der Formel (III-a), 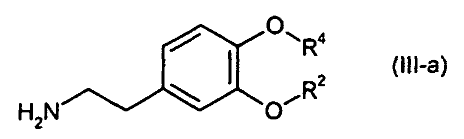 in welcher
R² für Alkoxyalkyl oder gegebenenfalls durch Methyl oder Halogen substituiertes Arylalkyl steht, und
R⁴ für Propargyl oder Cyanomethyl steht.

5. Verfahren zur Herstellung von Verbindungen der allgemeinen Formel (I), wie in Anspruch 1 definiert, **dadurch gekennzeichnet, dass** man Verbindungen der allgemeinen Formel (II), 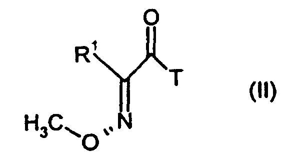 in welcher
R¹ die in Anspruch 1 angegebene Bedeutung hat und
T für Hydroxy, Halogen oder Alkoxy steht,
mit einem Amin der Formel (III), 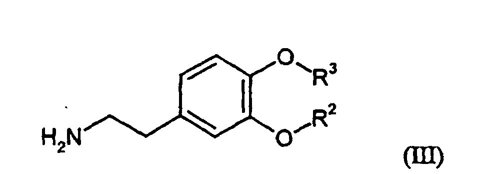 in welcher
R² und R³ die oben angegebenen Bedeutungen haben,
- oder mit einem Säureadditionskomplex hiervon -
gegebenenfalls in Gegenwart eines Säureakzeptors, gegebenenfalls in Gegenwart eines Kondensationsmittels und gegebenenfalls in Gegenwart eines Verdünnungsmittels umsetzt.

6. Mittel zur Bekämpfung von schädlichen Organismen, enthaltend Streckmittel und/oder Trägerstoffe sowie gegebenenfalls oberflächenaktive Stoffe, **gekennzeichnet durch** einen Gehalt von mindestens einer Verbindung wie in den Ansprüchen 1 bis 3 definiert.

7. Verfahren zur Bekämpfung von schädlichen Organismen, **dadurch gekennzeichnet, dass** man Verbindungen wie in den Ansprüchen 1 bis 3 bzw. Mittel wie in Anspruch 6 definiert auf schädliche Organismen und/oder ihren Lebensraum einwirken läßt.

8. Verwendung von Verbindungen wie in den Ansprüchen 1 bis 3 definiert bzw. von Mitteln wie in Anspruch 6 definiert zur Bekämpfung von Schädlingen.

9. Verfahren zur Herstellung von Mitteln wie in Anspruch 6 definiert, **dadurch gekennzeichnet, dass** man Verbindungen wie in den Ansprüchen 1 bis 3 definiert mit Streckmitteln und/oder Trägerstoffen und/oder oberflächenaktiven Mitteln vermischt.

## Claims

1. Compound of the general formula (I) 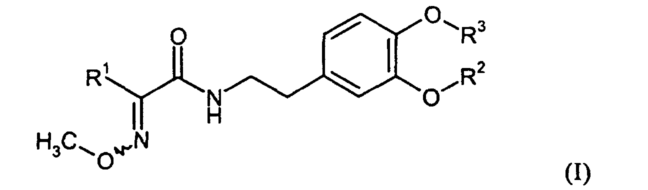 in which
R¹ represents aryl which optionally has a fused-on cycloalkyl ring, where both the aryl molecule and the cycloalkyl molecule optionally carry further substituents and
a)
R² represents alkoxyalkyl or optionally methyl- or halogen-substituted arylalkyl, and
R³ represents optionally substituted alkyl, alkenyl or alkinyl,
or
b)
R² represents optionally substituted alkyl, alkoxyalkyl or arylalkyl, and
R³ represents optionally substituted alkenyl,
or
c)
R² represents methyl or ethyl, and
R³ represents optionally substituted alkyl having at least 3 carbon atoms,
or
d)
R² represents alkyl having at least 2 carbon atoms, and
R³ represents optionally substituted methyl or ethyl.

2. Compound of the formula (I) according to Claim 1, **characterized in that**
R¹ represents aryl which optionally has a fused-on cycloalkyl ring having 3 to 10 ring members, where the cycloalkyl molecule is optionally substituted by 1 to 4 alkyl chains having in each case 1-4 carbon atoms and the aryl molecule is optionally additionally substituted by the substituents listed below:
Halogen, cyano, nitro, amino, hydroxyl, formyl, carboxyl, carbamoyl, thiocarbamoyl;
in each case straight-chain or branched alkyl, alkoxy, alkylthio, alkylsulfinyl or alkylsulfonyl having in each case 1 to 8 carbon atoms;
in each case straight-chain or branched alkenyl or alkenyloxy having in each case 2 to 6 carbon atoms;
in each case straight-chain or branched halogenalkyl, halogenalkoxy, halogenalkylthio, halogenalkylsulfinyl or halogenalkylsulfonyl having in each case 1 to 6 carbon atoms and 1 to 13 identical or different halogen atoms;
in each case straight-chain or branched halogenalkenyl or halogenalkenyloxy having in each case 2 to 6 carbon atoms and 1 to 13 identical or different halogen atoms;
in each case straight-chain or branched alkylamino, dialkylamino, alkylcarbonyl, alkylcarbonyloxy, alkoxycarbonyl, alkylsulfonyloxy, hydroximinoalkyl or alkoximinoalkyl having in each case 1 to 6 carbon atoms in the individual alkyl molecules;
cycloalkyl having 3 to 6 carbon atoms, and
a)
R² represents alkoxyalkyl having a total of 2 to 10 carbon atoms or optionally methyl- or halogen-substituted arylalkyl having 1 to 4 carbon atoms in the alkyl molecule and
R³ represents optionally cyano-, alkoxy- or alkoxycarbonyl-substituted alkyl having 1 to 8 carbon atoms, alkenyl or alkinyl,
or
b)
R² represents alkyl having 1 to 8 carbon atoms or optionally methyl- or halogen-substituted arylalkyl having 1 to 4 carbon atoms in the alkyl molecule and
R³ represents optionally cyano-, alkoxy- or alkoxycarbonyl-substituted alkenyl having 2 to 8 carbon atoms,
or
C)
R² represents methyl or ethyl, and
R³ represents optionally cyano-, alkoxy- or alkoxycarbonyl-substituted alkyl having at least 3 carbon atoms,
or
d)
R² represents alkyl having at least 2 carbon atoms, and
R³ represents optionally cyano-, alkoxy- or alkoxycarbonyl-substituted methyl or ethyl.

3. Compound of the formula (I) according to Claim 1, **characterized in that**
R¹ represents phenyl which is in each case optionally mono- to trisubstituted and which optionally has fused-on cyclopropyl, cyclobutyl, cyclopentyl, cyclohexyl, cycloheptyl, cylcooctyl or cyclononyl ring, where the cycloalkyl molecule is optionally mono- to tetrasubstituted by methyl, ethyl, n- or i-propyl, n-, i-, s- or t-butyl and the phenyl molecule is optionally additionally substituted by the substituents listed below:
Fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, n-pentyl, n-hexyl, n-heptyl, methoxy, ethoxy, n- or i-propoxy, methylthio, ethylthio, n- or i-propylthio, methylsulfinyl, ethylsulfinyl, methylsulfonyl or ethylsulfonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulfinyl or trifluoromethylsulfonyl, methylamino, ethylamino, n- or i-propylamino, dimethylamino, diethylamino, acetyl, propionyl, acetyloxy, methoxycarbonyl, ethoxycarbonyl, methylsulfonyloxy, ethylsulfonyloxy, hydroximinomethyl, hydroximinoethyl, methoximinomethyl, ethoximinomethyl, methoximinoethyl or ethoximinoethyl, cyclopropyl, cyclobutyl, cyclopentyl or cyclohexyl, and
a)
R² represents methoxyethyl, ethoxyethyl or optionally methyl-, fluorine- or chlorine-substituted benzyl or phenethyl and
R³ represents optionally cyano-, methoxy-, ethoxy-, methoxycarbonyl- or ethoxycarbonyl substituted methyl, ethyl, n- or i-propyl, n-, i-, s- or t-butyl, pentyl, hexyl, heptyl, octyl, allyl, methylallyl, crotonyl, propinyl, butinyl or benzyl,
or
b)
R² represents methyl, ethyl, n- or i-propyl, n-, i-, s- or t-butyl, pentyl, hexyl, heptyl or octyl or optionally methyl-, fluorine- or chlorine-substituted benzyl or phenethyl, and
R³ represents optionally cyano-, methoxy-, ethoxy-, methoxycarbonyl- or ethoxycarbonyl-substituted allyl, methylallyl, crotonyl,
or
c)
R² represents methyl or ethyl, and
R³ represents optionally cyano-, methoxy-, ethoxy-, methylcarbonyl or ethoxycarbonyl-substituted n- or i-propyl, n-, i-, s- or t-butyl, pentyl, hexyl, heptyl or octyl,
or
d)
R² represents n- or i-propyl, n-, i-, s- or t-butyl, pentyl, hexyl, heptyl or octyl, and
R³ represents optionally cyano-, methoxy-, ethoxy-, methoxycarbonyl- or ethoxycarbonyl-substituted methyl or ethyl.

4. Compound of the formula (III-a), 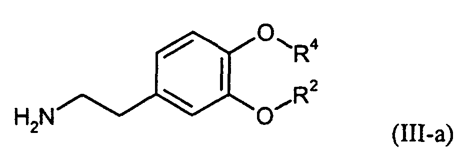 in which
R² is alkoxyalkyl, or optionally methyl- or halogen-substituted arylalkyl, and
R⁴ represents propargyl or cyanomethyl.

5. Process for preparing compounds of the general formula (I) as defined in Claim 1, **characterized in that** compounds of the general formula (II) 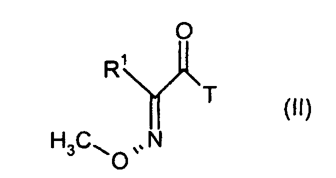 in which
R¹ is as defined in Claim 1 and
T represents hydroxyl, halogen or alkoxy,
are reacted with an amine of the formula (III), 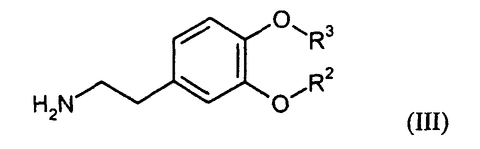 in which
R² and R³ are as defined above,
- or an acid addition complex thereof -
if appropriate in the presence of an acid acceptor, if appropriate in the presence of a condensing agent and if appropriate in the presence of a diluent.

6. Composition for controlling harmful organisms, which comprises extenders and/or carriers and also, if appropriate, surfactants, **characterized in that** it comprises at least one compound as defined in Claims 1 to 3.

7. Method for controlling harmful organisms, **characterized in that** compounds as defined in Claims 1 to 3 or compositions as defined in Claim 6 are allowed to act on harmful organisms and/or their habitat.

8. The use of compounds as defined in Claims 1 to 3 or of compositions as defined in Claim 6 for controlling pests.

9. Process for preparing compositions as defined in Claim 6, **characterized in that** compounds as defined in Claims 1 to 3 are mixed with extenders and/or carriers and/or surfactants.

## Revendications

1. Composés de la formule générale (I) : 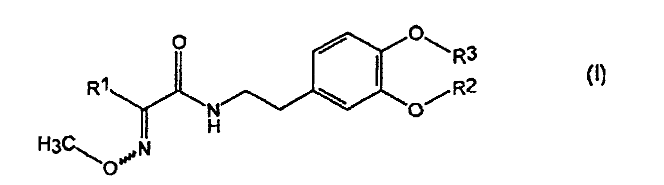 dans laquelle
R¹ représente un radical aryle, sur lequel est condensé le cas échéant, un radical cycloalcoyle, où non seulement la partie aryle, mais également la partie cycloalcoyle porte le cas échéant, d'autres substituants, et
a) R² représente un radical alcoxyalcoyle ou un radical arylalcoyle, le cas échéant substitué par le radical méthyle ou un atome d'halogène, et
R³ représente un radical alcoyle, alcényle
ou alcynyle le cas échéant substitué,
ou
b) R² représente un radical alcoyle, alcoxyalcoyle ou arylalcoyle, le cas échéant substitué, et
R³ représente un radical alcényle le cas échéant substitué,
ou
c) R² représente le radical méthyle ou éthyle, et
R³ représente un radical alcoyle le cas échéant substitué, avec au moins 3 atomes de carbone,
ou
d) R² représente un radical alcoyle avec au moins 2 atomes de carbone, et
R³ représente le radical méthyle ou éthyle le cas échéant substitué.

2. Composés de la formule (I), selon la revendication 1, **caractérisé en ce que** :
R¹ représente un radical aryle, sur lequel est condensé le cas échéant, un radical cycloalcoyle ayant 3 à 10 membres cycliques, où la partie cycloalcoyle est substituée le cas échéant, par 1 à 4 chaînes alcoyle avec chaque fois, 1 à 4 atomes de carbone, et la partie aryle est en outre substituée, le cas échéant, par les substituants énumérés ci-après :
un atome d'halogène, le radical cyano, nitro, amino, hydroxyle, formyle, carbonyle, carbamoyle, thiocarbamoyle ;
un radical alcoyle, alcoxy, alcoylthio, alcoylsulfinyle ou alcoylsulfonyle, chaque fois linéaire ou ramifié, avec chaque fois, 1 à 8 atomes de carbone ;
un radical alcényle ou alcényloxy, chaque fois linéaire ou ramifié, avec chaque fois, 2 à 6 atomes de carbone ;
un radical halogénoalcoyle, halogénoalcoxy, halogénoalcoylthio, halogénoalcoylsulfinyle ou halogénoalcoylsulfonyle, chaque fois linéaire ou ramifié, avec chaque fois, 1 à 6 atomes de carbone et 1 à 13 atomes d'halogène identiques ou différents ;
un radical halogénoalcényle ou halogéno-alcényloxy, chaque fois linéaire ou ramifié, avec chaque fois, 2 à 6 atomes de carbone et 1 à 13 atomes d'halogène identiques ou différents ;
un radical alcoylamino, dialcoylamino, alcoylcarbonyle, alcoylcarbonyloxy, alcoxycarbonyle, alcoylsulfonyloxy, hydroximinoalcoyle ou alcoximinoalcoyle, chaque fois linéaire ou ramifié, avec chaque fois, 1 à 6 atomes de carbone dans la partie alcoyle individuelle ;
un radical cycloalcoyle ayant 3 à 6 atomes de carbone, et
a) R² représente un radical alcoxyalcoyle avec au total, 2 à 10 atomes de carbone ou un radical arylalcoyle, le cas échéant substitué par le radical méthyle ou un atome d'halogène, avec 1 à 4 atomes de carbone dans la partie alcoyle, et
R³ représente un radical alcoyle ayant 1 à 8 atomes de carbone, alcényle ou alcynyle, le cas échéant substitué par le radical cyano, par un radical alcoxy ou alcoxycarbonyle,
ou
b) R² représente un radical alcoyle avec 1 à 8 atomes de carbone ou un radical arylalcoyle, le cas échéant substitué par le radical méthyle ou un atome d'halogène, avec 1 à 4 atomes de carbone dans la partie alcoyle, et
R³ représente un radical alcényle ayant 2 à 8 atomes de carbone, le cas échéant substitué par le radical cyano, par un radical alcoxy ou alcoxycarbonyle,
ou
c) R² représente le radical méthyle ou éthyle, et
R³ représente un radical alcoyle, le cas échéant substitué par le radical cyano, par un radical alcoxy ou alcoxycarbonyle, avec au moins 3 atomes de carbone,
ou
d) R² représente un radical alcoyle avec au moins 2 atomes de carbone, et
R³ représente un radical méthyle ou éthyle, le cas échéant substitué par le radical cyano, par un radical alcoxy ou alcoxycarbonyle.

3. Composés de la formule (I), selon la revendication 1, **caractérisé en ce que** :
R¹ représente le radical phényle, le cas échéant substitué une à trois fois, sur lequel est condensé le cas échéant, le radical cyclopropyle, cyclobutyle, cyclopentyle, cyclohexyle, cycloheptyle, cyclooctyle ou cyclononyle, où la partie cycloalcoyle est substituée le cas échéant, 1 à 4 fois, par le radical méthyle, éthyle, n- ou i-propyle, n-, i-, s- ou t-butyle, et la partie phényle est en outre substituée, le cas échéant, par les substituants énumérés ci-après :
l'atome de fluor, de chlore, de brome, le radical cyano, nitro, amino, hydroxyle, formyle, carboxyle, carbamoyle, thiocarbamoyle, le radical méthyle, éthyle, n- ou i-propyle, n-, i-, s- ou t-butyle, n-pentyle, n-hexyle, n-heptyle, méthoxy, éthoxy, n- ou i-propoxy, méthylthio, éthylthio, n- ou i-propylthio, méthylsulfinyle, éthylsulfinyle, méthylsulfonyle, éthylsulfonyle, trifluorométhyle, trifluoroéthyle, difluorométhoxy, trifluorométhoxy, difluorochlorométhoxy, trifluoroéthoxy, difluorométhylthio, difluorochlorométhylthio, trifluorométhylthio, trifluorométhylsulfinyle ou trifluorométhylsulfonyle, méthylamino, éthylamino, n- ou i-propylamino, diméthylamino, diéthylamino, acétyle, propionyle, acétyloxy, méthoxycarbonyle, éthoxycarbonyle, méthylsulfonyloxy, éthylsulfonyloxy, hydroxyminométhyle, hydroxyminoéthyle, méthoxyminométhyle, éthoxyminométhyle, méthoxyminoéthyle ou éthoximinoéthyle, cyclopropyle, cyclobutyle, cyclopentyle ou cyclohexyle, et
a) R² représente le radical méthoxyéthyle, éthoxyéthyle ou le radical benzyle ou phénéthyle, le cas échéant substitué par le radical méthyle, l'atome de fluor ou de chlore, et
R³ représente le radical méthyle, éthyle, n-ou i-propyle, n-, i-, s- ou t-butyle, pentyle, hexyle, heptyle, octyle, allyle, méthallyle, crotonyle, propynyle, butynyle ou benzyle, le cas échéant substitué par le radical cyano, méthoxy, éthoxy, méthoxycarbonyle ou éthoxycarbonyle,
ou
b) R² représente le radical méthyle, éthyle, n-ou i-propyle, n-, i-, s- ou t-butyle, pentyle, hexyle, heptyle, octyle, ou le radical benzyle ou phénéthyle, le cas échéant substitué par le radical méthyle, l'atome de fluor ou de chlore, et
R³ représente le radical allyle, méthallyle, crotonyle, le cas échéant substitué par le radical cyano, méthoxy, éthoxy, méthoxycarbonyle ou éthoxycarbonyle,
ou
c) R² représente le radical méthyle ou éthyle, et
R³ représente le radical n- ou i-propyle, n-, i-, s- ou t-butyle, pentyle, hexyle, heptyle, octyle, le cas échéant substitué par le radical cyano, méthoxy, éthoxy, méthoxycarbonyle ou éthoxycarbonyle,
d) R² représente le radical n- ou i-propyle, n-, i-, s- ou t-butyle, pentyle, hexylc, heptyle, octyle, et
R³ représente un radical méthyle ou éthyle, le cas échéant substitué par le radical cyano, méthoxy, éthoxy, méthoxycarbonyle ou éthoxycarbonyle.

4. Composés de la formule (III-a) : 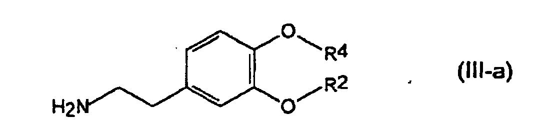 dans laquelle :
R² représente un radical alcoxyalcoyle ou un radical arylalcoyle le cas échéant substitué par le radical méthyle ou un atome d'halogène, et
R⁴ représente le radical propargyle ou cyanométhyle.

5. Procédé de préparation de composés de la formule générale (I), tels que définis à la revendication 1, **caractérisé en ce que** l'on fait réagir des composés de la formule générale (II) : 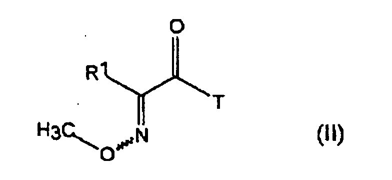 dans laquelle
R¹ a la signification indiquée à la revendication 1, et
T représente le radical hydroxyle, un atome d'halogène ou un radical alcoxy,
avec une amine de la formule (III) : 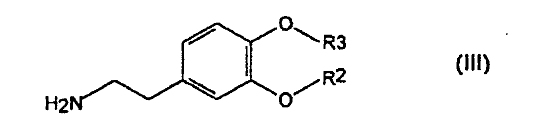 dans laquelle
R² et R³ ont les significations données ci-dessus,
ou avec un complexe d'addition d'acide de celle-ci,
le cas échéant en présence d'un accepteur d'acide, le cas échéant en présence d'un agent de condensation et le cas échéant, en présence d'un agent de dilution.

6. Agent pour lutter contre des organismes nuisibles, contenant un diluant et/ou une matière support, ainsi que le cas échéant, des substances tensioactives, **caractérisé par** une teneur en au moins un composé tel que défini dans les revendications 1 à 3.

7. Procédé pour lutter contre des organismes nuisibles, **caractérisé en ce que** l'on fait agir des composés tels que définis aux revendications 1 à 3 ou un agent tel que défini à la revendication 6, sur les organismes nuisibles et/ou leur biotope.

8. Utilisation des composés tels que définis aux revendications 1 à 3 ou de l'agent tel que défini à la revendication 6, pour lutter contre les parasites.

9. Procédé de préparation d'agents tels que définis dans la revendication 6, **caractérisé en ce que** l'on mélange les composés tels que définis aux revendications 1 à 3, avec un diluant et/ou une matière support et/ou des agents tensioactifs.
